Europäisches Patentamt

European Patent Office

Office européen des brevets

(11) Numéro de publication: **0 199 698 B1**

# (12) FASCICULE DE BREVET EUROPEEN

(45) Date de publication de fascicule du brevet:
**28.08.91**

(51) Int. Cl.5: **C12P 21/02**, C07K 3/00,
//C12N15/00

(21) Numéro de dépôt: **86870041.0**

(22) Date de dépôt: **02.04.86**

(54) **Procédé d'extraction et de purification de protéines issues de milieux de culture les produisant.**

(30) Priorité: **03.04.85 US 719601**

(43) Date de publication de la demande:
**29.10.86 Bulletin 86/44**

(45) Mention de la délivrance du brevet:
**28.08.91 Bulletin 91/35**

(84) Etats contractants désignés:
**AT BE CH DE FR GB IT LI LU NL SE**

(56) Documents cités:
EP-A- 0 106 828
US-A- 4 144 130

PROC.NATL.ACAD.SCI. USA, vol. 80, janvier 1983, pp. 1-5, Washington, US; A. MIYANOHA-RA et al.:"Expression of hepatitis B surface antigen gene in yeast"

NUCLEIC ACIDS RESEARCH, vol. 11, no. 9, mai 1983, pp. 2745-2763; Oxford, GB; R.A. HITZEMAN et al.:"Expression of hepatitis B virus surface antigen in yeast

(73) Titulaire: **SMITHKLINE BIOLOGICALS S.A.**
**Rue du Tilleul, 13**
**B-1320 Genval Rixensart(BE)**

(72) Inventeur: **van Wijnendaele, Frans**
**Teckerstraat, 12**
**B-3052 Ottenburg(BE)**
Inventeur: **Gilles, Daniel**
**Rue Mascau, 13**
**B-1320 Genval(BE)**
Inventeur: **Simonet, Guy**
**Chaussée de Wavre, 14**
**B-5920 Perwez(BE)**

(74) Mandataire: **Tyrrell, Arthur William Russell et al**
**SmithKline Beecham, Corporate Patents,**
**Great Burgh, Yew Tree Bottom Road**
**Epsom, Surrey KT18 5XQ(GB)**

ADVANCED HEPATITUS RESEARCH, 1984, chapitre 25, pp. 230-237, A.J. ZUCKER-MAN:"Alternative hepatitis B vaccines: polypeptide micelle vaccines"

JOURNAL OFVIROLOGICAL METHODS, vol. 14, 1986, pp. 25-35, Elsevier Sc. Publ. B.V., Amsterdam, NL, C.R. HOWARD, et al.:"Hepatitis B surface antigen polypeptide micelles from antigen expressed in Saccharomyces cerevisiae"

NATURE, vol. 290, 1981, pp. 51-54

## Description

Domaine de l'invention

La présente invention a pour objet un procédé perfectionné d'extraction et de purification de protéines issues de milieux de culture les produisant; plus particulièrement, le procédé concerne l'extraction et la purification de l'antigène de surface du virus de l'hépatite B et de l'alpha-l-antitrypsine produits par des techniques d'ADN recombinant dans des cultures cellulaires produites génétiquement, plus particulièrement des cultures de souches de levure produites génétiquement.

Fondement de l'invention

Divers microorganismes, parmi lesquels les bactéries et les levures, peuvent être utilisés comme organismes hôtes pour différents plasmides contenant une molécule d'ADN renfermant une séquence nucléotidique codant pour une protéine spécifique. Parmi ces microorganismes, les levures sont actuellement préférées et d'un emploi courant. Par exemple, la demande de brevet européen publiée sous le numéro 0 106 828 décrit l'utilisation des souches de Saccharomyces cerevisiae pour la production de l'antigène de surface du virus de l'hépatite B (HBsAg) et la demande de brevet européen publiée sous le numéro 0 103 409 est relative à la production de l'alpha-l-antitrypsine au départ de plasmides de levure.

La production de protéines dans des souches de levure présente, en effet, de nets avantages par rapport à la production dans des souches bactériennes. Ces avantages proviennent de la croissance plus aisée des levures dans des fermenteurs à grande échelle et du fait que, contrairement aux bactéries, les levures ressemblent assez bien aux cellules de mammifères pour leur capacité d'additionner des groupes glucidiques aux protéines qui viennent d'être synthétisées.

Toutefois, l'extraction et la purification de la protéine issue d'une culture de levure donnent lieu à des problèmes techniques dus à la composition chimique plutôt complexe de la cellule de levure et plus particulièrement à cause de la présence des taux lipidiques élevés lorsqu'on prolonge la croissance de la levure pour améliorer le rendement de la production en polypeptides.

Par exemple, la paroi de Saccharomyces est supposée se composer de 3 couches : (a) une couche interne de $\beta$-glucane insoluble en milieu alcalin, (b) une couche centrale de $\beta$-glucane alcalino-soluble et (c) une couche externe de glycoprotéine dans laquelle le glucide se compose de mannane phosphorylé; en dessous de la paroi se trouve une membrane cytoplasmique qui se compose d'un mélange très complexe constitué de lipides neutres (mono- , di- et triglycérides), de stérols libres et estérifiés, d'un sphingolipide complexe, de glycérophosphatides et de glycolipides neutres et acides; le noyau renferme de l'ADN, diverses espèces d'ARN et un polyphosphate; les vacuoles peuvent contenir une grande variété de composés de poids moléculaires élevés ou faibles; elles servent de vésicules de réserve destinées à de nombreuses hydrolases; les mitochondries sont riches en composés lipidiques, phospholipidiques et ergostéroliques en provenance du système membraneux et le cytoplasme contient entre autres de grandes quantités de ribosomes, de polyphosphates, de glycogènes et de nombreux enzymes de la glycolyse. La plupart des cellules de levure (telle que l'espèce Saccharomyces) contiennent également une certaine quantité de lipide sous la forme de globules dont la quantité augmente dans les cultures prolongées.

De nombreux procédés à étapes multiples ont été décrits pour l'extraction et la purification de protéines issues de différentes sources. Des exemples de production de protéines par des microorganismes obtenus génétiquement sont ceux publiés par Th. STAEHELIN et al. (J. Biol. Chem. 256:9750-54; 1981), K. MURRAY et al. (The Embo J. 3:645-650; 1984) et R.A. HITZEMAN et al. (Nucl. Ac. Res. 11:2745-2763; 1983).

En fait, lorsque la protéine produite est liée à la cellule, ces différents procédés impliquent 3 séries d'étapes.

Dans la première série d'étapes, la protéine désirée est isolée de l'intérieur de la cellule. A cette fin, les cellules sont soit lysées (p.ex. par traitement enzymatique) ou éclatées (p.ex. par des forces mécaniques telles que les forces de cisaillement (p.ex. la cellule de pression X ou la cellule de pression de French) ou agitées avec des billes de verre, avec addition éventuelle d'un détergent (voir par exemple K. MURRAY et al., loc. cit. et R.A. HITZEMAN et al., loc. cit.).

Dans la deuxième série d'étapes, le milieu est enrichi en protéine désirée, p.ex. par précipitation fractionnée par ajout de sulfate d'ammonium et/ou en présence de polyéthylène glycol.

Enfin, dans la troisième série d'étapes, tous les contaminants sont effectivement eliminés du milieu, p.ex. par une ou plusieurs opérations du genre ultrafiltration, chromatographie par échange d'ions et par filtration sur gel et centrifugation en gradient isopycnique.

Dans un tel procédé, il est évident que les contaminants accompagnant les protéines (cités par R.A. HITZEMAN et al., loc. cit.), les acides nucléiques et les lipides et plus particulièrement les taux de lipides élevés exercent une forte influence sur au moins une des étapes de la troisième série (p.e. l'ultrafiltration et la chromatographie sur colonne) et que les hydrolases doivent être rapidement éliminées du milieu. De plus, il a également été observé que la précipitation au sulfate d'ammonium n'est pas possible sans une délipidation préalable, étant donné que les lipides interfèrent dans cette précipitation.

En conséquence, il est de la première importance de pouvoir disposer d'une méthode par laquelle on puisse éliminer la plupart des contaminants dès le début de la troisième série d'étapes.

Parmi les procédés déjà décrits, certains citent l'utilisation du polyéthylène glycol comme agent de précipitation sélectif des protéines et un procédé de précipitation des lipoprotéines à partir du plasma en faisant appel aux interactions lipoprotéine-polyanionmétal a également été signalé.

La méthode de précipitation fractionnée des protéines à l'aide de polymères non ioniques hydrosolubles, en particulier le polyéthylène glycol (PEG) a été introduite par POLSON et al. (Biochem. Biophys. Acta 82:463-475; 1964) et discutée par divers auteurs. Parmi eux, W. HONIG et al. (Analyt. Biochem. 72:502-512; 1976) mentionnent que "la spécificité de la précipitation, c'est-à-dire le rapport de la protéine désirée et de la protéine totale peut être amélioré en utilisant des fractions de PEG d'un poids moléculaire moyen inférieur au PEG 6000 généralement employé". Néanmoins, quoique "des concentrés de protéines plasmatiques individuelles puissent être obtenus en agissant sur le pH", les auteurs ajoutent cependant que "la purification de mélanges protéiniques plus complexes tels que le surnageant d'un homogénat de cellule est considérablement plus médiocre".

P.R. FOSTER et al. (Biochim. Biophys. Acta 317: 505-516; 1973) ont décrit une méthode de précipitation d'enzymes à partir d'extraits cellulaires de Saccharomyces cerevisiae au moyen du PEG. Des méthodes de concentration et de purification de virus et de bactériophages à l'aide de PEG ont été publiées par B.P. VAJDA (Folia Microbiol. 23:88-96; 1978) et G.J. LANCZ (Arch. Virusforsch. 42:303-306; 1973). Dans le domaine de l'isolement de l'antigène de l'hépatite, la purification de l'antigène de surface de l'hépatite B (HBsAg) par une méthode comprenant deux traitements successifs avec le PEG 6000 a été décrite par Ph. ADAMOWICZ et al. (p. 37-49 INSERM SYMPOSIUM N° 18, HEPATITIS B VACCINE, Publ. ELSEVIER, Amsterdam, Holland, 1981). Dans cette méthode de purification de l'HBsAg au départ de plasma, les complexes immuns et la plupart des lipoprotéines sont, dans une première étape, précipités du sérum par le PEG 6000 à une concentration de 5,5 % et, dans une seconde étape, l'HBsAg est précipité du surnageant isolé par addition de PEG à une concentration finale de 10 %.

En ce qui concerne la littérature sur les brevets,
- le brevet des Etats-Unis d'Amérique N° 3790552 décrit une méthode d'élimination de l'antigène associé à l'hépatite à partir d'une fraction protéinique et qui comprend une étape dans laquelle on utilise du PEG ayant un poids moléculaire de 200 à 6000 à raison d'une quantité de 12 à 30 % (p/v) pour précipiter ledit antigène.
- le brevet des Etats-Unis d'Amérique N° 3951937 décrit un procédé de purification de l'antigène de l'hépatite B (HBAg) impliquant une double précipitation de l'HBAg au moyen de PEG (4 - 4,5 % en poids) ayant un poids moléculaire d'au moins 600.
- le brevet des Etats-Unis d'Amérique N° 3994870 décrit une méthode de purification de l'antigène de l'hépatite B (HBAg) dans laquelle l'HBAg est précipité par addition de 4 à 4,5 % en poids de PEG et est ensuite soumis à une chromatographie d'affinité à l'aide de concanavalline A comme adsorbant chromatographique.
- la demande de brevet européen publiée sous le numéro 0 112 506 décrit un procédé de production d'un vaccin préventif de l'infection à l'hépatite B à partir de plasma et qui comprend une précipitation au sulfate d'ammonium suivie d'adsorption sur du silicate colloïdal et de deux étapes de précipitations successives avec du PEG (ayant un poids moléculaire de 2000 à 10000) à raison de 3 à 7 % (p/v) de manière à précipiter le virus de l'hépatite B et les immuns complexes et à raison de 15 à 20 % (p/v) dans le surnageant de manière à précipiter l'HBsAg.
- Dans le domaine de l'isolement de l'alpha-l-antitrypsine, la demande de brevet japonais 59/128335 (résumé Derwent 84-217127) décrit la précipitation d'alpha-l-antitrypsine à partir d'une fraction plasmatique par addition d'une quantité de 15 à 20 % (p/v) de PEG.

Ainsi qu'il a été mentionné ci-dessus, une méthode de précipitation des lipoprotéines par application des interactions lipoprotéine-polyanion-métal a également été signalée précédemment (par exemple : M. BURSTEIN et al. Adv. Lip. Res., 11:67-108:1973 et A. VAN DALEN et al., Biochim. Biophys. Acta 147:421-427; 1967). Cette méthode est réalisée par interaction entre les lipoprotéines, un cation métallique bivalent et un polysaccharide acide et, dans ces conditions opératoires, la quantité de précipité est fonction de la concentration en cation métallique bivalent dans le milieu.

EP 0 199 698 B1

Description de l'invention

Le produit de départ utilisé dans le procédé de la présente invention (désigné également ici sous le nom d'"extrait brut") est le surnageant des cellules de levure produites génétiquement qui ont donné naissance à une protéine liée à la cellule et qui sont éclatées en présence d'un détergent non ionique, ainsi qu'il est bien connu dans la technique.

Conformément à la présente invention, on réalise alors la précipitation fractionnée par PEG (désignée ci-après sous le nom de première étape) et, ensuite, soit une précipitation fractionnée par un cation métallique polyvalent (plus particulièrement un métal bivalent tel que le calcium ou le manganèse) soit un traitement au sulfate d'ammonium, ledit traitement au sulfate d'ammonium étant éventuellement précédé d'une ultrafiltration du surnageant provenant de la première étape.

La présente invention résulte de la découverte que, lorsqu'on utilise du PEG solide (p.ex. PEG 6000) à une concentration de 6 à 12 % (p/v) pour extraire l'HBsAg à partir de cellules de levure produites génétiquement et éclatées en présence d'un détergent non ionique, l'HBsAg ne précipite pas. De plus, par addition ultérieure de sulfate d'ammonium, il se crée un système biphasé qui se caractérise par le fait que l'HBsAg est présent dans la phase PEG tandis que la majeure partie des contaminants se trouve dans la phase aqueuse. Ce résultat est surprenant étant donné que ces conditions opératoires devraient provoquer la précipitation de l'HBsAg en vertu de ce que l'art antérieur nous apprend généralement au sujet d'autres milieux bruts. Le premier objectif de la présente invention est donc d'utiliser le PEG comme solvant pour isoler la protéine désirée produite par des cultures de cellules de levure tandis que la majeure partie des contaminants sont éliminés par précipitation dans le milieu.

En ce qui concerne le poids moléculaire, le PEG existe soit sous forme solide, soit sous forme liquide, la limite entre les deux formes se situant autour d'un poids moléculaire de 1500.

Dans la première étape du procédé suivant la présente invention (qui pourrait être considérée également comme une étape de clarification), des PEGs ayant des poids moléculaires différents peuvent évidemment être utilisés en ajustant adéquatement leur concentration en fonction de leurs poids moléculaires respectifs.

Par exemple, la concentration en PEG varie, de préférence, de 6 à 12 % (p/v) lorsqu'on utilise du PEG solide (p.e. le PEG 6000) et de 10 à 35 % (v/v) - et de préférence de 20 à 30 % (v/v) lorsqu'on utilise du PEG liquide (p.e. le PEG 300 ou 400). Néanmoins, l'utilisation de PEG liquide est préférable pour des raisons techniques parmi lesquelles la facilité de la dernière ultrafiltration.

En conséquence, la présente invention décrit un procédé d'extraction et de purification de protéines, plus particulièrement de l'HBsAg ou de l'alpha-l-antitrypsine, au départ du surnageant de cellules de levure éclatées en présence d'un détergent non ionique (de préférence un détergent du type polysorbate), ledit surnageant étant désigné ici par "extrait brut", lequel procédé comprend une première étape dans laquelle on ajoute 6 à 12 % (p/v) de PEG solide ou de préférence 10 à 35 % (v/v) de PEG liquide à l'extrait brut amené à pH 6 (± 0,1), les limites des concentrations en PEG susmentionnées étant principalement déterminées par le moment où la clarification est atteinte puisqu'une addition supplémentaire de PEG ne produit que des effets défavorables, c.à.d. une viscosité plus élevée du milieu et le risque d'une coprécipitation inopportune d'HBsAg ou d'alpha-l-antitrypsine.

Dans le procédé de la présente invention, le PEG est considéré comme un solvant organique polymérisé favorisant e.a. la précipitation des (lipo)protéines dont le point isoélectrique se situe près du pH 6 et la solubilisation des autres (lipo)protéines, c.à.d. celles qui présentent un point isoélectrique nettement différent et celles qui sont fortement hydrophobes.

La demanderesse a remarqué que l'étape de clarification par le PEG précipite environ 75 % des protéines contaminantes, 90 % des polysaccharides, 94 % des acides nucléiques et 45 % des lipides, tandis que c'est le contenu total en HBsAg ou en alpha-l-antitrypsine qui est pratiquement récupéré dans le surnageant.

Ainsi qu'il a été indiqué ci-dessus, la présente invention comprend une combinaison d'étapes dont la première est une étape de clarification en vue d'obtenir une solution partiellement purifiée et éventuellement quelque peu opalescente de la protéine désirée.

Dans la deuxième étape du procédé de la présente invention, la solution obtenue et partiellement purifiée est traitée soit par un cation métallique polyvalent - plus particulièrement un cation métallique bivalent - comme une solution aqueuse de chlorure de calcium ou de manganèse à une concentration finale de 30 mM, soit par du sulfate d'ammonium, ce traitement au sulfate d'ammonium étant effectué soit après ultrafiltration de la solution opalescente par addition de sulfate d'ammonium solide jusqu'à 40 à 50 % de saturation, suivant la méthode classique dite de relargage pour précipiter la protéine désirée laquelle peut être reprise dans un tampon phosphate, soit par addition de sulfate d'ammonium au surnageant contenant

5

le PEG jusqu'à 40 à 50 % de saturation pour former un système biphasé contenant la protéine désirée dans la phase PEG.

Chacune de ces variations dans la deuxième série d'étapes donne lieu à une solution limpide en protéine désirée effectivement purifiée en ce qui concerne son contenu en polysaccharides, acides nucléiques, lipides et protéines contaminantes.

La solution ainsi obtenue peut ensuite être traitée dans la troisième série d'étapes classiques telle que définie ci-dessous, p.ex. ultrafiltration éventuelle, filtration sur gel et chromatographie sur colonne et, dans un mode de réalisation préféré de la présente invention, cette troisième série d'étapes comprend successivement une ultrafiltration, une première filtration sur gel, une chromatographie sur colonne échangeuse d'anion faiblement alcalin dotée de groupes diéthylaminoéthyle (DEAE) et une seconde filtration sur gel ou centrifugation sur gradient de chlorure de césium de manière à obtenir une solution d'HBsAg ou d'alpha-l-antitrypsine hautement purifiée et appropriée à un usage médical.

Lorsqu'on teste le produit isolé après la seconde perméation sur gel par électrophorèse sur gel de polyacrylamide sur dodécyl sulfate de sodium (SDS PAGE), on constate qu'il est pur à plus de 98 % en ce qui concerne la bande 23 K qui caractérise l'HBsAg issu de la levure.

En conséquence, le premier avantage du procédé de la présente invention réside dans le fait qu'il élimine effectivement tous les polysaccharides, les acides nucléiques, les lipides et les matières protéiniques et un autre avantage de la présente invention est que la protéine désirée peut finalement être séparée avec un rendement relativement élevé.

Lorsque le procédé de la présente invention est appliqué à l'extrait brut d'HBsAg en provenance des cellules de levure produites génétiquement, l'HBsAg obtenu est lié au détergent non ionique en formant avec celui-ci des micelles composites d'un diamètre d'environ 17 à 20 nm et la demanderesse a découvert que le rapport exprimant la quantité de détergent non ionique sur la quantité de protéines, de lipides totaux et de polysorbate dans la micelle composite de polysorbate varie de 15 à 35 % (p/p) lorsque les analyses sont effectuées par méthode colorimétrique pour les protéines (LOWRY), les lipides totaux (ZOLLNER) et le détergent non ionique (HUDDLESTON et ALLRED).

Les micelles composites obtenues par le procédé de la présente invention à l'aide d'un détergent non ionique de type polysorbate constituent des composés nouveaux qui font également l'objet de la présente invention; ils sont immunogènes et peuvent être présentés dans une forme vaccinale comparable à l'HBsAg classique ainsi qu'il est bien connu dans la technique pour la préparation du vaccin à base du virus de l'hépatite B.

L'invention est illustrée par les exemples suivants qui ne limitent en rien la porté de l'invention.

Exemple 1

Un culot de cellules de levure (3850 g) provenant d'une souche produite génétiquement et exprimant l'HBsAg et qui se sont développées jusqu'à l'obtention de 30 g de cellules en poids sec par litre de culture sont mises en suspension dans 7,12 litres d'une solution de $Na_2HPO_4$ (7,098 g/l).

Cette suspension est additionnée de 142,5 ml d'une solution de EDTA à 4 % (p/v), 38,5 ml de polysorbate 20 et 385 ml d'isopropanol contenant 2,7 g de fluorure de phénylméthylsulfonyle (FPMS). Le pH est ajusté à 8,1 (± 0,1) avec NaOH (10 % p/v dans l'eau). La suspension est refroidie dans un bain de glace et brisée par 2 passages à travers un homogénéiseur à billes de verre refroidi. L'homogénat (extrait brut) est ensuite centrifugé pendant 30 minutes à 13000 g.

On ajoute lentement, sous agitation, 2,5 1 de PEG 400 à 7,7 1 d'extrait brut que l'on maintient en dessous de 15° C. Le pH est ensuite ajusté à 6 (± 0,1) par addition d'acide acétique (5M) et le milieu est entreposé pendant une heure à 4° C avant d'être centri fugé à 7400-11000 g pendant 45 minutes.

Le surnageant est amené à pH 7 (± 0,05) avec NaOH N et on y ajoute 300 ml de $CaCl_2$ (c.à.d. 30 ml par litre de surnageant). Le pH est réajusté à 7 (± 0,05) avec NaOH N et entreposé à 4° C jusqu'au lendemain. La suspension est centrifugée à 3600 g pendant 45 minutes et le surnageant est ultrafiltré dans un appareil AMICON DC (appareil vendu par AMICON CORP., DANVERS, MA, USA) équipé d'une cartouche ayant un cut-off de 100000 daltons et, par ce moyen, il est d'abord concentré jusqu'à 1/4 (1500 ml) de son volume initial. La solution est ensuite lavée de façon continue avec 12,5 ml de trométamol 10mM ajusté à pH 8 (± 0,1) par addition d'acide chlorhydrique N (ce tampon est désigné ci-après par trométamol pH 8) et additionné de PMSF (1 mM), d'isopropanol (2,5 % v/v) et d'EDTA (2 mM) (concentrations finales). Le rétentat est ensuite concentré ultérieurement jusqu'à 350 ml).

La solution concentrée est versée sur une colonne (diamètre 10 cm x 100 cm) contenant 7 1 de FRACTOGEL[R] TSK HW65(F) (un gel semi-rigide composé de polymères vinyliques hydrophiles pourvus de nombreux groupes hydroxyles à la surface de la matrice, présentant une dimension de particules de l'ordre de 32 à 63 μm, fabriqué et vendu par E. Merck, Darmstadt, RFA) équilibré dans un tampon trométamol/HCl 10 mM pH 7 (± 0,01) additionné de 5 % (v/v) d'éthylène glycol.

Le pic contenant l'HBsAg est versé sur une colonne (diamètre 5 cm x 30 cm) de 300 ml de FRACTOGEL[R] TSK DEAE 650 (M) (un échangeur anionique faiblement basique dans lequel les groupes diéthylaminoéthyle sont liés aux groupes hydroxyle de la matrice du FRACTOGEL TSK HW65 par des liaisons éthérées, fabriqué et vendu par E. Merck, Darmstadt, RFA) équilibré dans du trométamol pH 8 à 4° C. La colonne est lavée avec du trométamol pH 8 contenant NaCl (0,05 M) et l'HBsAg est élué avec NaCl (0,15 M) dans du trométamol pH 8.

L'éluat est appliqué sur une colonne de FRACTOGEL TSK HW65 (F) équilibrée avec un tampon $Na_2HPO_4/NaH_2PO_4$ (10 mM) pH 6,8 additionné de NaCl (150 mM) pour donner une solution de micelles composites du type HBsAg/polysorbate.

Le taux de purification obtenu à chaque étape de la purification est indiqué au tableau I.

## TABLEAU I

| Fraction | Vol (1) | Total en HBsAg par RIE (mg) | Total en protéi- nes (g) | Total en polysac- charides (g) | Total en acides nucléi- ques (mg) | Total en lipides (g) |
|---|---|---|---|---|---|---|
| Extrait brut | 7,7 | 1232 | 259 | 114 | 32500 | 104 |
| Surna- geant PEG | 8,02 | 1100 | 64 | 9,5 | 641 | 53,7 |
| Surna- geant $CaCl_2$ | 8 | 1190 | 37 | 3,8 | 512 | 27,2 |
| Rétentat | .350 | 1320 | 26,7 | .744 | 37,6 | 3,596 |
| Pic HBsAg TSK Eluat | 1,06 | 736 | .935 | .014 | 16 | .352 |
| TSK-DEAE Pic HBsAg | .15 | 722 | .428 | .0084 | 2,6 | .275 |
| TSK | .45 | 1117 | .244 | .0094 | 2 | .179 |

RI3 : Radio-immuno essai

Le tableau II suivant résumé sommairement la composition des micelles composites de 3 lots différents de vaccins obtenus par le procédé ci-dessus avec du polysorbate 20.

TABLEAU II

| Lot | Protéines (µg/ml) (A) | Total en lipides (µg/ml) (B) | Polysorbate 20 (ug/ml) (C) | Rapport $\frac{C}{A/B/C}$ |
|-----|------|------|------|------|
| I | 20 | 20 | 8,5 | 18 |
| II | 20 | 16 | 8,4 | 19 |
| III | 20 | 14,5 | 15,6 | 31 |

Exemple 2

On ajoute lentement 882 ml de PEG 400 à 2,650 l d'extrait brut préparé comme il est décrit à l'exemple 1 et on ajuste le pH à 6 ($\pm$ 0,1). Le milieu est maintenu à 4° C pendant une heure et centrifugé ensuite à 7400 g. Le surnageant est concentré à 1000 ml sur un appareil AMICON DC équipé d'une cartouche ayant un cut-off de 100000 daltons et lavé ensuite avec 3 volumes de trométamol (20 mM) pH 8. Sous agitation et à 4° C, on ajoute ensuite lentement 277 g de sulfate d'ammonium en poudre au rétentat. Après 1 heure à 4° C, le précipité est centrifugé pendant 15 min. à 1000 g. Le surnageant est écarté et le précipité est dissous dans 400 ml de trométamol (20 mM) pH 8 additionné de 1 mM de PMSF. La solution est ultrafiltrée sur un appareil AMICON DC équipé d'une cartouche ayant un cut-off de $10^6$ daltons. Le rétentat (500 ml) est lavé avec 2 volumes de trométamol pH 8 et appliqué ensuite sur une colonne contenant 300 ml de FRACTOGEL TSK DEAE 650M équilibré avec du trométamol pH 8. Lorsque le passage de l'échantillon est achevé, la colonne est lavée avec NaCl 0,05 M dans du trométamol pH 8 et un gradient linéaire en NaCl (0,05 M - 0,5 M) est ensuite appliqué sur la colonne. La fraction éluée avec le NaCl 0,15 M contient l'HBsAg: elle est concentrée à 50 ml dans un appareil AMICON DC équipé d'une cartouche ayant un cut-off de 10000 daltons et appliquée sur une colonne (diamètre 50 x 100 cm) de Sepharose 4B-Cl (un gel d'agarose fabriqué et vendu par Pharmacia Fine Chemicals, Uppsala, Suède) équilibrée avec du trométamol (20 0M) pH 8 additionné de 5 % d'éthylène glycol pour donner une solution de micelles composites de type HBsAg/polysorbate.

Le taux de purification obtenu à chaque étape est indiqué au tableau III.

EP 0 199 698 B1

## TABLEAU III

| Fraction | Vol (ml) | Total en HBsAg par RIE (mg) | Total en protéines (g) | Total en polysaccharides (g) | Total en acides nucléiques (mg) |
|---|---|---|---|---|---|
| Extrait brut | 2650 | 188 | 114,721 | 36,9 | 9250 |
| Surnageant PEG | 2530 | 160 | 22,755 | 3,321 | 536 |
| Rétentat $10^5$ | 1000 | 150 | 7,468 | .181 | 175 |
| AMS-culot | 400 | 80,8 | 1,248 | .0248 | 160 |
| Rétentat $10^6$ | 1000 | 72,4 | .529 | .029 | 88 |
| Eluat TSK-DEAE Pic HBsAg | 560 | 44 | .157 | <0,073 | 3 |
| Sépharose 4B-CL | 200 | 23 | .030 | .0003 | .05 |

RIE : Radio-immuno essai

Exemple 3

On clarifie 2 litres d'extrait brut d'HBsAg avec du PEG 400 comme il est décrit à l'exemple 1. On y ajoute 450 g de sulfate d'ammonium (SAM) en poudre sous agitation (la concentration finale correspond à une saturation à 45 % en SAM. Après solubilisation du SAM, la solution est conservée à 4° C pendant une période de 3 heures au terme de laquelle on obtient deux phases distinctes que l'on sépare dans une ampoule à décantation : une phase supérieure limpide (jaune) (phase PEG 400) et une phase inférieure limpide (phase aqueuse), chacune des phases représentant environ ± 50 % du volume original. La phase aqueuse est éliminée et la phase supérieure est ultrafiltrée sur un appareil AMICON DC, comme il est décrit pour le surnageant du $CaCl_2$ à l'exemple 1. Le processus de purification est ensuite poursuivi ultérieurement comme il est décrit à l'exemple 1 pour donner une solution de micelles composites de type HBsAg/polysorbate.

Le taux de purification obtenu à chaque étape est indiqué au tableau IV.

## TABLEAU IV

| Fraction | Vol (ml) | Total en HBsAg par RIE (mg) | Total en protéi- nes (g) | Total en polysac- charides (g) | Total en acides nucléi- ques (mg) |
|---|---|---|---|---|---|
| Extrait brut | 2000 | 99 | 55 | 22 | 5588 |
| Surnageant PEG | 2000 | 92 | 13,2 | 5,68 | 243 |
| phase PEG | 1000 | 98 | 2,8 | 1,536 | 98 |
| Retentat | 50 | 98 | 1,5 | .14 | 6,66 |
| Pic HBsAg TSK | 123 | 52 | .288 | .0077 | 2,84 |
| Eluat TSK-DEAE | 25 | 54 | .0377 | .0011 | .44 |
| Pic HBsAg TSK | 60 | 35 | .0207 | .00154 | .39 |

RIE : Radio-immuno essai

### Exemple 4

On clarifie 2 litres d'extrait brut en HBsAg avec du PEG 400 comme il est décrit à l'exemple 1. On y ajoute 450 g de sulfate d'ammonium (SAM) en poudre sous agitation (la concentration finale correspond à une saturation à 45 % en SAM). Après solubilisation du SAM, la solution est conservée à 4° C pendant une période de 3 heures au terme de laquelle on obtient deux phases distinctes que l'on sépare dans une ampoule à décantation : une phase supérieure limpide (jaune) (phase PEG 400) et une phase inférieure limpide (phase aqueuse), chacune des phases représentant environ ± 50 % du volume original. La phase aqueuse est éliminée et une partie aliquote d'un litre de la phase PEG (phase supérieure) est diluée deux fois avec du trométamol 10 mM à pH 8 et appliquée sur une colonne contenant 300 ml de gel du type FRACTOGEL TSK-DEAE 650 (M) équilibrée avec du trométamol à pH 8; à raison d'un débit de 100 ml par heure. Le gel est lavé avec du trométamol à pH 8 contenant NaCl 0,05 M et l'HBsAg est ensuite élué avec un gradient en NaCl (NaCl 0,05 à 0,5 M dans le trométamol à pH 8). Un pic HBsAg est élué avec NaCl 0,15 M et après concentration dans un appareil AMICON DC équipé d'une cartouche ayant un cut-off de 10000 daltons, le rétentat est appliqué sur une colonne (diamètre 5 x 100 cm) de Sépharose 4B-Cl équilibrée avec du trométamol pH 8 contenant 5 % (v/v) d'éthylène glycol pour donner un pic de micelles composites de type HBsAg/polysorbate/

Le taux de purification obtenu à chaque étape est indiqué au tableau V.

## TABLEAU V

| Fraction | Vol | Total en HBsAg par RIE | Total en protéines | Total en polysaccharides | Total en acides nucléiques |
|---|---|---|---|---|---|
| | (ml) | (mg) | (g) | (g) | (mg) |
| Extrait brut | 2100 | 200 | 60,600 | 19,000 | 4860 |
| phase PEG | 1200 | 210 | 3,100 | 1,436 | 80 |
| Eluat TSK-DEAE | 460 | 134 | .750 | .0048 | 05 |
| Pic HBsAg/ Sépharose 4B-CL | 150 | 110 | .120 | .0014 | 0.3 |

RIE : Radio-immuno essai

### Exemple 5

On clarifie 500 ml d'extrait brut en HBsAg par addition lente d'une solution de 50 g de PEG 6000 dans 160 ml d'eau à 4° C, sous agitation. On ajuste le pH à 6,1 par addition d'acide acétique 5M. Après une heure de stockage à 4° C, l'extrait est centrifugé pendant 15 min. à 7000 g. On ajoute ensuite, sous agitation, 157 g de sulfate d'ammonium en poudre au surnageant (la concentration finale correspond à 50 % de sulfate d'ammonium saturé). Après solubilisation, la solution est conservée à 4° C pendant une période de 3 heures au terme de laquelle on obtient deux phases distinctes que l'on sépare dans une ampoule à décantation. La phase supérieure (phase PEG contenant l'HBsAg) est diluée deux fois avec du trométamol à pH 8 et appliquée ensuite sur une colonne contenant 300 ml de gel de TSK-DEAE 650 (M) équilibrée avec du trométamol ) pH 8 à raison d'un débit de 100 ml par heure. Le gel est lavé avec du trométamol à pH 8 contenant NaCl 0,15 M, concentré dans un appareil AMICON DC équipé d'une cartouche ayant un cut-off de 10000 daltons et le rétentat est appliqué ensuite sur une colonne (diamètre 5 x 100 cm) de 2 litres de FRACTOGEL TSK-HW65(F) équilibré avec du trométamol à pH 8 contenant 10 % (v/v) d'éthylène glycol pour donner une solution de micelles composites de type HBsAg/polysorbate.

Le taux de purification à chaque étape est indiqué au tableau VI.

## TABLEAU VI

| Fraction | Vol | Total en HBsAg par RIE | Total en protéines | Total en polysaccharides | Total en acides nucléiques |
|---|---|---|---|---|---|
| | (ml) | (mg) | (g) | (g) | (mg) |
| Extrait brut | 500 | 123 | 22,600 | 6,400 | 1200 |
| Surnageant PEG | 490 | 100 | 4,153 | .509 | 24 |
| phase PEG | 270 | 110 | 1,820 | .2096 | 5,3 |
| Eluat TSK-DEAE | 150 | 66 | .258 | .028 | .4 |
| Pic HBsAg TSK | 60 | 68 | .065 | .00048 | .084 |

RIE : Radio-immuno essai

Exemple 6

On ajoute lentement 13 ml de PEG 400 à 120ml d'extrait brut en alpha-1-antitrypsine provenant d'une culture de levure produite génétiquement. Après une heure de stockage à 4° C, le milieu est centrifugé à 5000 g pendant 15 minutes et on y ajoute lentement une solution de $CaCl_2$ M jusqu'à une concentration finale de 40 mM. Le pH est ajusté à 7 avec NaOH M et après un stockage à 4° C jusqu'au lendemain, le milieu est centrifugé à 7000 g pendant 15 minutes. Le surnageant est dilué deux fois avec du trométamol 50 mM ajusté à pH 8,7 par addition d'acide chlorhydrique 0,1 N et appliqué sur une colonne de FRACTOGEL TSK-DEAE 650(M) (20 ml) équilibré avec du trométamol 50 mM à pH 8,7 préparé comme il est indiqué ci-dessus mais additionné de mercaptoéthanol 5 mM. La colonne est lavée avec du trométamol à pH 8,7 comme il est indiqué ci-dessus mais additionné de NaCl 10 mM, un gradient en NaCl est appliqué (10 mM-250 mM) et l'alpha-l-antitrypsine est éluée à une concentration en Nacl de 124 mM, séparée d'un pic protéinique élué à une concentration en NaCl de 80 mM. Le pic en alpha-l-antitrypsine est concentré dans un appareil AMICON DC équipé d'une cartouche ayant un cut-off de 10000 daltons et appliqué sur une colonne de Sephadex 150 équilibrée avec du trométamol 50 mM à pH 8,7, préparé comme il est indiqué ci-dessus, pour donner une solution d'alpha-l-antitrypsine purifiée.

Le taux de purification obtenu à chaque étape est indiqué au tableau VII.

## TABLEAU VII

| Fraction | Vol | Total en AAT par ELISA | Total en protéines | Total en polysaccharides |
| --- | --- | --- | --- | --- |
| | (ml) | (mg) | (g) | (g) |
| Extrait brut | 120 | 180 | 4,018 | .538 |
| Surnageant PEG | 100 | 212 | 1,300 | .290 |
| Surnageant CaCl$_2$ | 100 | 194 | .660 | .110 |
| Eluat TSK-DEAE | 520 | 180 | .210 | .0052 |

AAT : alpha-1-antitrypsine

Exemple 7

La technique est telle que décrite à l'exemple 1, mais on remplace les 300 ml de CaCl$_2$ M qui y sont spécifiés par 300 ml de MnCl$_2$ M. Les caractéristiques du produit final sont analogues à celles du produit obtenu à la fin de l'exemple 1.

Exemple 8

La technique est telle que décrite à l'exemple 1, mais on remplace les 38,5 ml de polysorbate 20 qui y sont spécifiés par 20 ml de Triton X-100 (un produit fabriqué par Rohm et Haas; Darmstadt, RFA). Les caractéristiques du produit final sont analogues à celles du produit obtenu à la fin de l'exemple 1.

Exemple 9

La technique est telle que décrite à l'exemple 1, mais on remplace les 38,5 ml de polysorbate 20 qui y sont spécifiés par 38,5 ml de polysorbate 80. Les caractéristiques du produit final sont analogues à celles du produit obtenu à la fin de l'exemple 1.

Exemple 10

La solution du micelle composite de type HBsAg obtenu à la fin de l'exemple 1 est ajustée à un contenu protéinique de 10 μg par millilitre par addition de NaCl, de tampon phosphate (Na$_2$HPO$_4$/NaH$_2$PO$_4$) et d'ALHYDROGEL$^R$ (un gel d'hydroxyde d'aluminium fabriqué et vendu par SUPERHOS Export Co. Copenhague, Danemark) jusqu'à des concentrations finales respectives de 0,9 % (p/v), 20 mM et 0,15 % (p/v) en Al(OH)$_3$, le pH final étant de 6,9. La préparation est stérilisée et répartie dans des fioles en verre de 2 ml contenant chacune une does unitaire d'un ml de vaccin.

Exemple 11

Les doses unitaires du vaccin de l'exemple 10 ont été administrées par voie intramusculaire à deux séries de sujets séronégatifs à raison de 2 injections successives, à un mois d'intervalle. Bien que ces injections devraient être considérées comme une administration de sensibilisation qui devrait être suivie d'un rappel, p.ex. 2 mois après la première injection, une réponse immunitaire positive a déjà été observée respectivement un et deux mois après la première administration.

13

Dans la première série (comprenant 32 sujets séronégatifs), un mois après la première administration, le taux de séroconversion était de 20/32, c'est-à-dire de 62,5 % avec un titre géométrique moyen (TGM) de 9.95 unités milli-internationales (UMI) et un mois après la seconde administration, le taux de séroconversion était de 31/32, c'est-à-dire de 96,9 % avec un TGM de 36 UMI.

Dans la seconde série (comprenant 46 sujets séronégatifs), un mois après la première administration, le taux de séroconversion était de 31/46, c'est-à-dire de 67,4 % avec un TGM de 13,6 UMI.

L'enregistrement des signes et symptômes cliniques chez les vaccinés n'a révélé aucune élévation de température et aucune réaction à l'endroit de l'administration.

## Revendications
### Revendications pour les Etats contractants suivants : BE, CH, DE, FR, GB, IT, LI, LU, NL, SE

1. Procédé d'extraction et de purification d'une protéine liée à la cellule à partir du surnageant d'une culture de cellules de levure manipulées génétiquement ayant produit ladite protéine et éclatées en présence d'un détergent non ionique, caractérisé en ce qu'on ajuste le pH du surnageant à 6 (± 0,1), on y ajoute du polyéthylène glycol liquide ou solide jusqu'à clarification dudit surnageant et on traite le surnageant clarifié soit par un cation métallique bivalent, soit, après ultrafiltration éventuelle, par du sulfate d'ammonium pour séparer ladite protéine.

2. Procédé suivant la revendication 1, dans lequel on ajoute du polyéthylène glycol liquide à une concentration finale comprise entre 10 et 35 % (v/v).

3. Procédé suivant la revendication 2, dans lequel on ajoute du polyéthylène glycol solide à une concentration finale comprise entre 6 et 12 % (p/v).

4. Procédé suivant l'une quelconque des revendications 1 à 3, dans lequel la protéine est l'antigène de surface de l'hépatite B.

5. Procédé suivant l'une quelconque des revendications 1 à 3, dans lequel la protéine est l'alpha-l-antitrypsine.

6. Procédé suivant l'une quelconque des revendications 1 à 5, dans lequel le surnageant clarifié est traité par un cation métallique bivalent.

7. Procédé suivant la revendication 6, dans lequel le cation métallique bivalent est le cation calcium ou manganèse.

8. Procédé suivant l'une quelconque des revendications 6 et 7, qui comprend en outre la séparation du précipité, l'ultrafiltration de la solution, la perméation sur gel du rétentat, la chromatographie sur échangeurs d'ions du pic contenant la protéine et éventuellement une seconde perméation sur gel ou une centrifugation sur gradient de chlorure de cesium.

9. Procédé suivant l'une quelconque des revendications 1 à 5, dans lequel on traite le surnageant clarifié par du sulfate d'ammonium jusqu'à 40 à 50 % de saturation et on isole la protéine purifiée dans la phase polyéthylène glycol.

10. Procédé suivant la revendication 9, dans lequel la phase polyéthylène glycol est soumise en outre à une éventuelle ultrafiltration suivie d'une perméation sur gel et d'une chromatographie sur échangeurs d'ions.

11. Procédé suivant la revendication 9, dans lequel la phase polyéthylène glycol est soumise en outre à une éventuelle ultrafiltration suivie d'une chromatographie sur échangeurs d'ions et d'une perméation sur gel.

12. Procédé suivant la revendication 2, dans lequel le surnageant clarifié est soumis à une ultrafiltration et le rétentat est traité par du sulfate d'ammonium jusqu'à 40 à 50 % de saturation afin de précipiter la protéine qui est reprise dans un tampon adéquat.

13. Procédé suivant la revendication 12 qui comprend en outre l'ultrafiltratration, la perméation sur gel et la chromatographie sur échangeurs d'ions.

14. Procédé suivant l'une quelconque des revendications 1 à 5, dans lequel le détergent non ionique est un polysorbate.

15. Procédé suivant la revendication 14, dans lequel le polysorbate est le polysorbate 20.

16. Micelle composite à base d'antigène de surface de l'hépatite B et de polysorbate contenant de 15 à 35 % (p/p) de polysorbate.

17. Vaccin contre l'hépatite B contenant à titre d'ingrédient actif une micelle composite suivant la revendication 16.

**Revendications pour l'Etat contractant suivant : AT**

1. Procédé d'extraction et de purification d'une protéine liée à la cellule à partir du surnageant d'une culture de cellules de levure manipulées génétiquement ayant produit ladite protéine et éclatées en présence d'un détergent non ionique, caractérisé en ce qu'on ajuste le pH du surnageant à 6 (± 0,1), on y ajoute du polyéthylène glycol liquide ou solide jusqu'à clarification dudit surnageant et on traite le surnageant clarifié soit par un cation métallique bivalent, soit, après ultrafiltration éventuelle, par du sulfate d'ammonium pour séparer ladite protéine.

2. Procédé suivant la revendication 1, dans lequel on ajoute du polyéthylène glycol liquide à une concentration finale comprise entre 10 et 35 % (v/v).

3. Procédé suivant la revendication 2, dans lequel on ajoute du polyéthylène glycol solide à une concentration finale comprise entre 6 et 12 % (p/v).

4. Procédé suivant l'une quelconque des revendications 1 à 3, dans lequel la protéine est l'antigène de surface de l'hépatite B.

5. Procédé suivant l'une quelconque des revendications 1 à 3, dans lequel la protéine est l'alpha-l-antitrypsine.

6. Procédé suivant l'une quelconque des revendications 1 à 5, dans lequel le surnageant clarifié est traité par un cation métallique bivalent.

7. Procédé suivant la revendication 6, dans lequel le cation métallique bivalent est le cation calcium ou manganèse.

8. Procédé suivant l'une quelconque des revendications 6 et 7, qui comprend en outre la séparation du précipité, l'ultrafiltration de la solution, la perméation sur gel du rétentat, la chromatographie sur échangeurs d'ions du pic contenant la protéine et éventuellement une seconde perméation sur gel ou une centrifugation sur gradient de chlorure de cesium.

9. Procédé suivant l'une quelconque des revendications 1 à 5, dans lequel on traite le surnageant clarifié par du sulfate d'ammonium jusqu'à 40 à 50 % de saturation et on isole la protéine purifiée dans la phase polyéthylène glycol.

10. Procédé suivant la revendication 9, dans lequel la phase polyéthylène glycol est soumise en outre à une éventuelle ultrafiltration suivie d'une perméation sur gel et d'une chromatographie sur échangeurs d'ions.

11. Procédé suivant la revendication 9, dans lequel la phase polyéthylène glycol est soumise en outre à une éventuelle ultrafiltration suivie d'une chromatographie sur échangeurs d'ions et d'une perméation sur gel.

**12.** Procédé suivant la revendication 2, dans lequel le surnageant clarifié est soumis à une ultrafiltration et le rétentat est traité par du sulfate d'ammonium jusqu'à 40 à 50 % de saturation afin de précipiter la protéine qui est reprise dans un tampon adéquat.

**13.** Procédé suivant la revendication 12 qui comprend en outre l'ultrafiltratration, la perméation sur gel et la chromatographie sur échangeurs d'ions.

**14.** Procédé suivant l'une quelconque des revendications 1 à 5, dans lequel le détergent non ionique est un polysorbate.

**15.** Procédé suivant la revendication 14, dans lequel le polysorbate est le polysorbate 20.

**Claims**
**Claims for the following Contracting States : BE, CH, DE, FR, GB, IT, LI, LU, NL, SE**

**1.** A process for the extraction and purification of a protein, bound to the cell, from the supernatant of a culture of genetically manipulated yeast cells which have produced said protein and burst in the presence of a non-ionic detergent, characterized in that the pH of the supernatant is adjusted to 6 (±0.1), liquid or solid polyethylene glycol is added until said supernatant has been clarified, and the clarified supernatant is treated either with a divalent metal cation or, after ultrafiltration if appropriate, with ammonium sulphate in order to separate out said protein.

**2.** A process according to claim 1 in which liquid polyethylene glycol is added up to a final concentration of between 10 and 35% (v/v).

**3.** A process according to Claim 2 in which solid polyethylene glycol is added up to a final concentration of between 6 and 12% (w/v).

**4.** A process according to any one of Claims 1 to 3 in which the protein is hepatitis B surface antigen.

**5.** A process according to any one of Claims 1 to 3 in which the protein is alpha-1-antitrypsin.

**6.** A process according to any one of Claims 1 to 5 in which the clarified supernatant is treated with a divalent metal cation.

**7.** A process according to Claim 6 in which the divalent metal cation is the calcium or manganese cation.

**8.** A process according to Claim 6 or claim 7 which also comprises separation of the precipitate, ultrafiltration of the solution, gel permeation of the retained material, ion exchange chromatography of the peak containing the protein and, if appropriate, a second gel permeation or centrifugation on a caesium chloride gradient.

**9.** A process according to any one of Claims 1 to 5 in which the clarified supernatant is treated with ammonium sulphate up to 40 to 50% of the saturation level and the purified protein is isolated from the polyethylene glycol phase.

**10.** A process according to Claim 9 in which the polyethylene glycol phase is also subjected to ultrafiltration if appropriate, followed by gel permeation and ion exchange chromatography.

**11.** A process according to Claim 9 in which the polyethylene glycol phase is also subjected to ultrafiltration if appropriate, followed by ion exchange chromatography and gel permeation.

**12.** A process according to Claim 2 in which the clarified supernatant is subjected to ultrafiltration and the retained material is treated with ammonium sulphate up to 40 to 50% of the saturation level in order to precipitate the protein, which is taken up in an appropriate buffer.

**13.** A process according to Claim 12 which also comprises ultrafiltration, gel permeation and ion exchange chromatography.

16

**14.** A process according to any one of Claims 1 to 5 in which the non-ionic detergent is a polysorbate.

**15.** A process according to Claim 14 in which the polysorbate is polysorbate 20.

**16.** A composite micelle based on hepatitis B surface antigen and polysorbate and containing from 15 to 35% (w/w) of polysorbate.

**17.** A hepatitis B vaccine containing, as the active ingredient, a composite micelle according to Claim 16.

**Claims for the following Contracting State : AT**

**1.** A process for the extraction and purification of a protein, bound to the cell, from the supernatant of a culture of genetically manipulated yeast cells which have produced said protein and burst in the presence of a non-ionic detergent, characterized in that the pH of the supernatant is adjusted to 6 (±0.1), liquid or solid polyethylene glycol is added until said supernatant has been clarified, and the clarified supernatant is treated either with a divalent metal cation or, after ultrafiltration if appropriate, with ammonium sulphate in order to separate out said protein.

**2.** A process according to Claim 1 in which liquid polyethylene glycol is added up to a final concentration of between 10 and 35% (v/v).

**3.** A process according to Claim 2 in which solid polyethylene glycol is added up to a final concentration of between 6 and 12% (w/v).

**4.** A process according to any one of Claims 1 to 3 in which the protein is hepatitis B surface antigen.

**5.** A process according to any one of Claims 1 to 3 in which the protein is alpha-1-antitrypsin.

**6.** A process according to any one of Claims 1 to 5 in which the clarified supernatant is treated with a divalent metal cation.

**7.** A process according to Claim 6 in which the divalent metal cation is the calcium or manganese cation.

**8.** A process according to Claim 6 or Claim 7 which also comprises separation of the precipitate, ultrafiltration of the solution, gel permeation of the retained material, ion exchange chromatography of the peak containing the protein and, if appropriate, a second gel permeation or centrifugation on a caesium chloride gradient.

**9.** A process according to any one of Claims 1 to 5 in which the clarified supernatant is treated with ammonium sulphate up to 40 to 50% of the saturation level and the purified protein is isolated from the polyethylene glycol phase.

**10.** A process according to Claim 9 in which the polyethylene glycol phase is also subjected to ultrafiltration if appropriate, followed by gel permeation and ion exchange chromatography.

**11.** A process according to Claim 9 in which the polyethylene glycol phase is also subjected to ultrafiltration if appropriate, followed by ion exchange chromatography and gel permeation.

**12.** A process according to Claim 2 in which the clarified supernatant is subjected to ultrafiltration and the retained material is treated with ammonium sulphate up to 40 to 50% of the saturation level in order to precipitate the protein, which is taken up in an appropriate buffer.

**13.** A process according to Claim 12 which also comprises ultrafiltration, gel permeation and ion exchange chromatography.

**14.** A process according to any one of Claims 1 to 5 in which the non-ionic detergent is a polysorbate.

**15.** A process according to Claim 14 in which the polysorbate is polysorbate 20.

**Patentansprüche**
**Patentansprüche für folgende Vertragsstaaten : BE, CH, DE, FR, GB, IT, LI, LU, NL, SE**

1. Verfahren zur Extraktion und Reinigung eines an die Zelle gebundenen Proteins aus dem Überstand einer Kultur von genetisch manipulierten Hefezellen, die das Protein erzeugt haben und die in Gegenwart eines nicht-ionischen Detergents aufgebrochen worden sind, **dadurch gekennzeichnet,** daß man den pH-Wert des Überstandes auf 6 (± 0,1) einstellt, flüssiges oder festes Polyethylenglykol bis zur Klärung des Überstandes zusetzt und den geklärten Überstand zur Abtrennung des Proteins entweder mit einem zweiwertigen Metall-Kation oder, gegebenenfalls nach Ultrafiltration, mit Ammoniumsulfat behandelt.

2. Verfahren nach Anspruch 1, wobei man flüssiges Polyethylenglykol bis zu einer Endkonzentration zwischen 10 und 35 Vol. -% zusetzt.

3. Verfahren nach Anspruch 2, wobei man festes Polyethylenglykol bis zu einer Endkonzentration zwischen 6 und 12 Gew.-/Vol. -% zusetzt.

4. Verfahren nach einem der Ansprüche 1 bis 3, wobei das Protein Hepatitis B-Oberflächenantigen ist.

5. Verfahren nach einem der Ansprüche 1 bis 3, wobei das Protein $\alpha$-1-Antitrypsin ist.

6. Verfahren nach einem der Ansprüche 1 bis 5, wobei der geklärte Überstand mit einem Zweiwertigen Metall-Kation behandelt wird.

7. Verfahren nach Anspruch 6, wobei das zweiwertige MetallKation das Calcium- oder Magnesium-Kation ist.

8. Verfahren nach einem der Ansprüche 6 und 7, umfassend ferner Abtrennung des Niederschlags, Ultrafiltration der Lösung, Gelpermeation des Retentats, Ionenaustauscherchromatographie des das Protein enthaltenden Peaks und schließlich eine zweite Gelpermeation oder eine Zentrifugation auf einem Cäsiumchlorid-Gradienten.

9. Verfahren nach einem der Ansprüche 1 bis 5, wobei man den geklärten Überstand mit Ammoniumsulfat bis zu 40 bis 50 % Sättigung behandelt und das gereinigte Protein in der Polyethylenglykolphase isoliert.

10. Verfahren nach Anspruch 9, wobei die Polyethylenglykolphase ferner gegebenenfalls einer Ultrafiltration und anschließend einer Gelpermeation und einer Ionenaustauschchromatographie unterzogen wird.

11. Verfahren nach Anspruch 9, wobei die Polyethylenglykolphase ferner gegebenenfalls einer Ultrafiltration und dann einer Ionenaustauschchromatographie und einer Gelpermeation unterzogen wird.

12. Verfahren nach Anspruch 2, wobei der geklärte Überstand einer Ultrafiltration unterzogen und das Retentat zur Fällung des Proteins mit Ammoniumsulfat bis zu 40 bis 50 % Sättigung behandelt und das Protein in einem geeigneten Puffer aufgenommen wird.

13. Verfahren nach Anspruch 12, umfassend ferner Ultrafiltration, Gelpermeation und Ionenaustauschchromatographie.

14. Verfahren nach einem der Ansprüche 1 bis 5, wobei das nicht-ionische Detergents ein Polysorbat ist.

15. Verfahren nach Anspruch 14, wobei das Polysorbat Polysorbat 20 ist.

16. Micellen-Zusammensetzung auf der Basis von Hepatitis BOberflächenantigen und Polysorbat, enthaltend 15 bis 35 Gew.-% Polysorbat.

17. Impfstoff gegen Hepatitis B, enthaltend als Wirkstoff eine Micellen-Zusammensetzung nach Anspruch 16.

**Patentansprüche für folgenden Vertragsstaat : AT**

1. Verfahren zur Extraktion und Reinigung eines an die Zelle gebundenen Proteins aus dem Überstand einer Kultur von genetisch manipulierten Hefezellen, die das Protein erzeugt haben und die in Gegenwart eines nicht-ionischen Detergents aufgebrochen worden sind, **dadurch gekennzeichnet,** daß man den pH-Wert des Überstandes auf 6 (± 0,1) einstellt, flüssiges oder festes Polyethylenglykol bis zur Klärung des Überstandes zusetzt und den geklärten Überstand zur Abtrennung des Proteins entweder mit einem zweiwertigen Metall-Kation oder, gegebenenfalls nach Ultrafiltration, mit Ammoniumsulfat behandelt.

2. Verfahren nach Anspruch 1, wobei man flüssiges Polyethylenglykol bis zu einer Endkonzentration zwischen 10 und 35 Vol.-% zusetzt.

3. Verfahren nach Anspruch 2, wobei man festes Polyethylenglykol bis zu einer Endkonzentration zwischen 6 und 12 Gew.-/Vol.-% zusetzt.

4. Verfahren nach einem der Ansprüche 1 bis 3, wobei das Protein Hepatitis B-Oberflächenantigen ist.

5. Verfahren nach einem der Ansprüche 1 bis 3, wobei das Protein $\alpha$-1-Antitrypsin ist.

6. Verfahren nach einem der Ansprüche 1 bis 5, wobei der geklärte Überstand mit einem zweiwertigen Metall-Kation behandelt wird.

7. Verfahren nach Anspruch 6, wobei das zweiwertige Metall-Kation das Calcium- oder Magnesium-Kation ist.

8. Verfahren nach einem der Ansprüche 6 und 7, umfassend ferner Abtren-nung des Niederschlags, Ultrafiltration der Lösung, Gelpermeation des Retentats, Ionenaustauscherchromatographie des das Protein enthaltenden Peaks und schließlich eine zweite Gelpermeation oder eine Zentrifugation auf einem Cäsiumchlorid-Gradienten.

9. Verfahren nach einem der Ansprüche 1 bis 5, wobei man den geklärten Überstand mit Ammoniumsulfat bis zu 40 bis 50 % Sättigung behandelt und das gereinigte Protein in der Polyethylenglykolphase isoliert.

10. Verfahren nach Anspruch 9, wobei die Polyethylenglykolphase ferner gegebenenfalls einer Ultrafiltration und anschließend einer Gelpermeation und einer Ionenaustauschchromatographie unterzogen wird.

11. Verfahren nach Anspruch 9, wobei die Polyethylenglykolphase ferner gegebenenfalls einer Ultrafiltration und dann einer Ionenaustauschchromatographie und einer Gelpermeation unterzogen wird.

12. Verfahren nach Anspruch 2, wobei der geklärte Überstand einer Ultrafiltration unterzogen und das Retentat zur Fällung des Proteins mit Ammoniumsulfat bis zu 40 bis 50 % Sättigung behandelt und das Protein in einem geeigneten Puffer aufgenommen wird.

13. Verfahren nach Anspruch 12, umfassend ferner Ultrafiltration, Gelpermeation und Ionenaustauschchromatographie.

14. Verfahren nach einem der Ansprüche 1 bis 5, wobei das nicht-ionische Detergents ein Polysorbat ist.

15. Verfahren nach Anspruch 14, wobei das Polysorbat Polysorbat 20 ist.